Europäisches Patentamt

European Patent Office

Office européen des brevets

(19)

(11) Publication number: **0 085 544**
**A1**

# (12) EUROPEAN PATENT APPLICATION

(21) Application number: **83300430.2**

(22) Date of filing: **27.01.83**

(51) Int. Cl.³: **A 61 K 31/19**
**//C07C57/30, C07C57/58**

(30) Priority: **01.02.82 US 344442**
**01.02.82 US 344443**
**01.02.82 US 344444**
**01.02.82 US 344704**

(43) Date of publication of application:
**10.08.83 Bulletin 83/32**

(84) Designated Contracting States:
**BE CH DE FR GB IT LI NL SE**

(71) Applicant: **THE UPJOHN COMPANY**
**301 Henrietta Street**
**Kalamazoo, Michigan 49001(US)**

(72) Inventor: **Lomen, Pavel Luboslav**
**c/o The Upjohn Company 301 Henrietta Street**
**Kalamazoo Michigan 49001(US)**

(74) Representative: **Perry, Robert Edward et al,**
**GILL JENNINGS & EVERY 53-64 Chancery Lane**
**London WC2A 1HN(GB)**

(54) The use of aralkanoic acids and esters for the treatment of myelogenous leukemia.

(57) Flurbiprofen and ibuprofen, and their $C_{1-8}$ alkyl esters and pharmacologically accpetable salts, are of utility in the treatment of acute or chronic myelogenous leukemia, e.g. in humans, particularly in association with a subsequent administration of a cytotoxic agent such as 5-azacytidine.

EP 0 085 544 A1

Croydon Printing Company Ltd.

## THE USE OF ARALKANOIC ACIDS AND ESTERS FOR THE TREATMENT OF MYELOGENOUS LEUKEMIA

This invention relates to the use of aralkanoic acid and esters in the treatment of myelogenous leukemia.

Myelogenous leukemia, a non-lymphocytic leukemia, occurs in children and adults in two forms, acute and chronic. Various treatment regimens have been used, using cytotoxic agents for chemotherapeutic treatment. These cytotoxic agents are, for example, busulfan, chlorambucil, 6-mercaptopurine, vincristine, doxorubicin, cytarabine, methotrexate and 5-azacytidine. Combinations of these cytotoxic agents have been used, in various sequences, for example, as disclosed in "Treatment of Acute Myelogenous Leukemia in Children and Adults", Weinstein et al., The New England Journal of Medicine, 303, no. 9, August 28, 1980, pp. 473-478. Dosage regimens using a single cytotoxic agent have been used, as disclosed in Cancer, 47, no. 7, April 1, 1981, pp. 1739-1742 and Annals of Internal Medicine, 85 (1976) pp. 237-245.

Flurbiprofen and ibuprofen are non-steroidal antiinflammatory drugs (NSAID) which have been used in rheumatic and degenerative diseases of the joints and for reducing platelet adhesiveness.

The active compounds of the present invention are flurbiprofen (3-fluoro-4-phenylhydratropic acid) and ibuprofen (p-isobutylhydratropic acid), their $C_{1-8}$ alkyl esters and pharmacologically acceptable salts.

The esters can be the methyl, ethyl, propyl, isopropyl, butyl, isobutyl, tert-butyl, pentyl, and octyl esters.

Pharmacologically acceptable salts can be, for example, the alkali metal, alkaline earth, and ammonium salts.

The compositions of the present invention are preferably presented for systemic administration to humans and animals in unit dosage forms, such as tablets, capsules, pills, powders, granules, suppositories, sterile parenteral solutions or suspensions, sterile non-parenteral solutions or suspensions, and oral solutions or suspensions and the like, containing suitable quantities of an active ingredient.

For oral administration either solid or fluid unit dosage forms can be prepared.

Powders are prepared quite simply by comminuting the active ingredient to a suitable fine size and mixing with a similarly comminuted diluent. The diluent can be an edible carbohydrate material such as lactose or starch. Advantageously, a sweetening agent or sugar is present as well as a flavoring oil.

Capsules are produced by preparing a powder mixture as hereinafter described and filling into formed gelatin sheaths. Advantageously, as an adjuvant to the filling operation, a lubricant such as talc, magnesium stearate, calcium stearate and the like is added to the powder mixture before the filling operation.

Soft gelatin capsules are prepared by machine encapsulation of a slurry of active ingredients with an acceptable vegetable oil, light liquid petrolatum or other inert oil or triglyceride.

Tablets are made by preparing a powder mixture, granulating or slugging, adding a lubricant and pressing into tablets. The powder mixture is prepared by mixing an active ingredient, suitably comminuted, with a diluent or base such as starch, lactose, kaoline, dicalcium phosphate and the like. The powder mixture can be granulated by wetting with a binder such as corn syrup, gelating solution, methylcellulose solution or acacia mucilage and forcing through a screen. As an alternative to granulating, the powder mixture can be slugged, i.e., run through the tablet machine and the resulting imperfectly formed tablets broken into pieces (slugs). The slugs can be lubricated to prevent sticking to the tablet-forming dies by means of the addition of stearic acid, a stearic salt, talc or mineral oil. The lubricated mixture is then compressed into tablets.

Advantageously, the tablet can be provided with a protective coating consisting of a sealing coat or enteric coat of shellac, a coating of sugar and methylcellulose and a polish coating of carnauba wax.

Fluid unit dosage forms for oral administration such as syrups, elixirs and suspensions can be prepared wherein each teaspoonful of composition contains a predetermined amount of active ingredient for administration. The water-soluble forms can be dissolved in an aqueous vehicle together with sugar, flavoring agents and preservatives to form a syrup. An elixir is prepared by using a hydroalcoholic vehicle with suitable sweeteners together with a flavoring agent. Suspensions can be prepared of the insoluble forms with a suitable vehicle with the aid of a suspending agent such as acacia, tragacanth, methylcellulose and the like.

For parenteral administration, fluid unit dosage forms are prepared utilizing an active ingredient and a sterile vehicle, water being preferred. The active ingredient, depending on the form and concentration used, can be either suspended or dissolved in the vehicle. In preparing solutions the water-soluble active ingredient can be dissolved in water for injection and filter sterilized before filling into a suitable vial or ampule and sealing. Advantageously, adjuvants such as a local anesthetic, preservative and buffering agents can be dissolved in the vehicle. Parenteral suspensions are prepared in substantially the same manner except that an active ingredient is suspended in the vehicle instead of being dissolved and sterilization cannot be accomplished by filtration. The active ingredient can be sterilized by exposure to ethylene oxide before suspending in the sterile vehicle. Advantageously, a surfactant or wetting agent is included in the composition to facilitate uniform distribution of the active ingredient.

The term "unit dosage form" as used in the specification and claims refers to physically discrete units suitable as unitary dosages for humans and animal subjects, each unit containing a predetermined quantity of active material calculated to produce the desired therapeutic effect in association with the required pharmaceutical diluent, carrier or vehicle. The specification for the novel unit dosage forms of this invention are dictated by and are directly dependent on (a) the unique characteristics of the active material and the particular therapeutic effect to be achieved, and (b) the limitation inherent in the art of compounding such an active material for therapeutic use in humans, as disclosed in this specification, these being features of the present invention. Examples of suitable unit dosage forms in accord with this invention are tablets, capsules, troches, suppositories, powder packets, wafers, cachets, teaspoonfuls, tablespoonfuls, dropperfuls, ampules, vials, segregated multiples of any of the foregoing, and other forms as herein described.

The systemic administration of flurbiprofen or ibuprofen, their salts or esters in conjunction with the treatment of acute or chronic myelogenous leukemia in adults and children provides a method of enhance induction of complete remission, prolong remission time or reduce toxicity to non-cancerous cells of the bone marrow, precursor cells or skin cells.

The dose of flurbiprofen, its salts or esters, for treating acute or chronic myelogenous leukemia according to the present invention is the same dose known for treating conditions for which it is previously known to be useful. In general, from about 2.5 mg to about 5 mg per kilogram body weight is administered daily in single or divided dosage amounts.

For remission induction of acute myelogenous leukemia using a single cytotoxic agent (e.g., 5-azacytidine), flurbiprofen is administered for 1-2 days followed by a drug free interval of 6 hours after which a single cytotoxic agent is administered for 12 hours. After this a drug free interval of 18 hours is followed by another cycle of flurbiprofen and cytotoxic agent. The number of cycles administered is determined by evaluation of the status of bone marrow during the 18 hours drug free interval. When complete remission occurs, flurbiprofen is administered daily, at a dose of 200 mg per day until 2 days prior to recommencement of administration of cytotoxic agent.

The flurbiprofen when used for remission induction in conjunction with a regimen of multiple cytotoxic agents, is not administered on days when the cyctotoxic agents are administered but rather an intermittant schedule is followed. The flurbiprofen is administered for 3 days at 300 mg/day (average adult) then no drug is administered for 1 day and then chemotherapy with cytotoxic agents is commenced. When cytotoxic agents are stopped and remission has been accomplished, the flurbiprofen is administered daily at 200 mg/day (average adult) until 2 days prior to recommencement of administration of cytotoxic agents used for maintenance chemotherapy.

The dose of ibuprofen, its salts or esters, for treating acute or chronic myelogenous leukemia according to the present invention is the same dose known for treating conditions for which it is previously known to be useful. In general, from about 25 mg to about 50 mg per kilogram body weight is administered daily in single or divided dosage amounts. For children about 30 mg/kg is used.

For remission induction of acute myelogenous leukemia using a single cytotoxic agent (e.g., 5-azacytidine), ibuprofen is administered for 1-2 days followed by a drug free interval of 6 hours after which a single cytotoxic agent is administered for 12 hours. After this a drug free interval of 18 hours is followed by another cycle of ibuprofen and cytotoxic agent. The number of cycles administered is determined by evaluation of the status of bone marrow during the 18 hours drug free interval. When complete remission occurs, ibuprofen is administered daily, at a dose of 2000 mg per day until 2 days prior to recommencement of administration of cytotoxic agent.

The ibuprofen when used for remission induction in conjunction with a regimen of multiple cytotoxic agents, is not administered on days when the cytotoxic agents are administered but rather an intermittant schedule is followed. The ibuprofen is administered for 3 days at 2400 mg per day (average adult) then no drug is administered for 1 day and then chemotherapy with cytotoxic agents is commenced. When cytotoxic agents are stopped and remission has been accomplished, the ibuprofen is administered daily at 1600 mg per day (average adult) until 2 days prior to recommencement of adminstration of cytotoxic agents used for maintenance chemotherapy.

The course of the disease is followed and determined by bone marrow biopsy, i.e., diagnosis of the disease, induction of remission, state of remission and relapse.

The following examples are illustrative of the present invention, but are not intended to be limiting.

Example 1    Hard Gelatin Capsules

One thousand two-piece hard gelatin capsules for oral use, each capsule containing 100 mg of flurbiprofen are prepared from the following types and amounts of ingredients:

Flurbiprofen                    100 gm

| | |
|---|---|
| Lactose | 100 gm |
| Corn starch | 20 gm |
| Talc | 20 gm |
| Magnesium stearate | 2 gm |

The flurbiprofen (finely divided by means of an air micronizer) is added to the other finely powdered ingredients, mixed thoroughly and then encapsulated in the usual manner.

The foregoing capsules are useful for remission induction of acute myelogenous leukemia in adults by the administration of 1 capsule 3 times a day for 2 days, followed by 6 hours of drug free interval, followed by 12 hours of administration of single cytotoxic agent and followed with 18 hours of drug free interval. The foregoing cycles repeated until complete remission occurs.

Using the procedure above, capsules are similarly prepared containing flurbiprofen in 10, 25, and 50 mg amounts by substituting 10, 25, and 50 gm of flurbiprofen for the 100 gm used above.

Example 2    Soft Gelatin Capsules

One-piece soft gelatin capsules for oral use, each containing 100 mg of flurbiprofen (finely divided by means of an air micronizer) are prepared by first suspending the compound in 0.5 ml of corn oil to render the material capsulatable and then capsulating in the above manner.

The foregoing capsules are used for treating acute myelogenous leukemia in adults by the administration regimen of Example 1.

Example 3    Tablets

One thousand tablets, each containing 100 mg of flurbiprofen are prepared from the following types and amounts of ingredients:

| | |
|---|---|
| Flurbiprofen | 100 gm |
| Lactose | 75 gm |
| Corn starch | 50 gm |
| Magnesium stearate | 4 gm |
| Light liquid petrolatum | 5 gm |

The flurbiprofen (finely divided by means of an air micronizer) is added to the other ingredients and then thoroughly mixed and slugged. The slugs are broken down by forcing through a number sixteen screen. The resulting granules are then compressed into tablets, each tablet containing 100 mg of flurbiprofen.

The foregoing tablets are used in remission induction of acute myelogenous leukemia by administering 1 tablet orally every 6 hours for 18 hours, followed by a drug free interval of 6 hours after which 5-azacytidine is administered parenterally for 12 hours. After this a drug free interval of 18 hours is followed by another cycle of flurbiprofen and the 5-azacytidine. The number of cycles administered is determined by the status of bone marrow during the 18 hours drug free interval. When complete remission occurs flurbiprofen is administered daily, at a dose of 200 mg per day until 2 days prior to recommencement of the administration of cytotoxic agent used for maintenance chemotherapy.

Example 4    Oral Suspension

One thousand ml of an aqueous suspension for oral use, containing in each teaspoonful (5 ml) dose, 100 mg of flurbiprofen, aluminum salt is prepared from the following types and amounts of ingredients:

| | |
|---|---|
| Flurbiprofen, aluminum salt | |
| micronized | 20 gm |
| Citric acid | 2 gm |
| Benzoic acid | 1 gm |
| Sucrose | 700 gm |
| Tragacanth | 5 gm |
| Lemon oil | 2 gm |
| Deionized water, q.s. | 1000 ml |

The citric acid, benzoic acid, sucrose, tragacanth and lemon oil are dispersed in sufficient water to make 850 ml of suspension. The flurbiprofen aluminum salt (finely divided by means of an air micronizer) is stirred into the syrup until uniformly distributed. Sufficient water is added to make 1000 ml.

The oral composition so prepared is useful in remission maintenance by the administration of 1 teaspoonful twice a day.

Example 5

A sterile aqueous solution for parenteral (i.v.) injection, containing in 300 ml, 150 mg of flurbiprofen, sodium salt is prepared from the following types and amounts of ingredients:

| | |
|---|---|
| Flurbiprofen sodium salt | 150 mg |
| Water for injection, q.s. | 300 ml |

To the sterile solution is added sterilized flurbiprofen, sodium salt and filled into sterile containers sealed.

The composition so prepared is useful for treating acute myelogenous leukemia in children by administration of 300 ml every 12 hours for 2 days, followed by drug free interval of 6 hours, followed by an induction course of treatment with 5-azacytidine until remission is induced. When remission is induced flurbiprofen is given orally on a daily basis during remission.

Example 6   Hard Gelatin Capsules

One thousand two-piece hard gelatin capsules for oral use, each capsule containing 100 mg of flurbiprofen are prepared from the following types and amounts of ingredients:

| Flurbiprofen | 100 gm |
| Lactose | 100 gm |
| Corn starch | 20 gm |
| Talc | 20 gm |
| Magnesium stearate | 2 gm |

The flurbiprofen (finely divided by means of an air micronizer) is added to the other finely powdered ingredients, mixed thoroughly and then encapsulated in the usual manner.

The foregoing tablets are used in remission maintenance of chronic myelogenous leukemia in adults by administering 200 mg/day commencing upon termination of treatment with cytotoxic agents and recovery of platelet count to 100,000/cmm. and terminating 2 days prior to recommencing treatment with cytotoxic agents

Using the procedure above, capsules are similarly prepared containing flurbiprofen in 10, 25, and 50 mg amounts by substituting 10, 25, and 50 gm of flurbiprofen for the 100 gm used above.

Example 7   Soft Gelatin Capsules

One-piece soft gelatin capsules for oral use, each containing 100 mg of flurbiprofen (finely divided by means of an air micronizer) are prepared by first suspending the compound in 0.5 ml of corn oil to render the material capsulatable and then capsulating in the above manner.

The foregoing capsules are used in remission maintenance of chronic myelogenous leukemia by administering 200 mg/day commencing upon termination of treatment with cytotoxic agents and recovery of platelet count to 100,000/cmm. and terminating 2 days prior to recommencing treatment with cytotoxic agents.

Example 8   Tablets

One thousand tablets, each containing 100 mg of flurbiprofen are prepared from the following types and amounts of ingredients:

| | |
|---|---|
| Flurbiprofen | 100 gm |
| Lactose | 75 gm |
| Corn starch | 50 gm |
| Magnesium stearate | 4 gm |
| Light liquid petrolatum | 5 gm |

The flurbiprofen (finely divided by means of an air micronizer) is added to the other ingredients and then thoroughly mixed and slugged. The slugs are broken down by forcing through a number sixteen screen. The resulting granules are then compressed into tablets, each tablet containing 100 mg of flurbiprofen.

The foregoing tablets are used in remission maintenance of chronic myelogenous leukemia by administering 200 mg/day commencing upon termination of treatment with cytotoxic agents and recovery of platelet count to 100,000/cmm. and terminating 2 days prior to recommencing treatment with cytotoxic agents.

Example 9  Oral Suspension

One thousand ml of an aqueous suspension for oral use, containing in each teaspoonful (5 ml) dose, 100 mg of flurbiprofen, aluminum salt is prepared from the following types and amounts of ingredients:

| | |
|---|---|
| Flurbiprofen, aluminum salt | |
| micronized | 20 gm |
| Citric acid | 2 gm |
| Benzoic acid | 1 gm |
| Sucrose | 700 gm |
| Tragacanth | 5 gm |
| Lemon oil | 2 gm |
| Deionized water, q.s. | 1000 ml |

The citric acid, benzoic acid, sucrose, tragacanth and lemon oil are dispersed in sufficient water to make 850 ml of suspension. The flurbiprofen aluminum salt (finely divided by means of an air micronizer) is stirred into the syrup until uniformly distributed. Sufficient water is added to make 1000 ml.

The oral composition so prepared is useful in remission maintenance by the administration of 1 teaspoonful twice a day.

Example 10

A sterile aqueous solution for parenteral (i.v.) injection, containing in 500 ml, 100 mg of flurbiprofen, sodium salt is prepared from the following types and amounts of ingredients:

| | |
|---|---|
| Flurbiprofen sodium salt | 100 mg |
| Water for injection, q.s. | 500 ml |

To the sterile solution is added sterilized flurbiprofen, sodium salt and filled into sterile containers sealed.

The composition so prepared is useful for treating acute myelogenous leukemia in children by administration of 500 ml every 12 hours for 3 days, followed by 1 day of drug free interval, followed by an induction course of treatment with cytotoxic agent until remission is induced. When remission is induced flurbiprofen is given orally on a daily basis during remission as 100-200 mg/day until 2 days prior to recommencement of administration of cytotoxic agents used for maintenance chemotherapy.

Example 11 Hard Gelatin Capsules

One thousand two-piece hard gelatin capsules for oral use, each capsule containing 600 mg of ibuprofen are prepared from the following types and amounts of ingredients:

| | |
|---|---|
| Ibuprofen | 600 gm |
| Lactose | 100 gm |
| Corn starch | 20 gm |
| Talc | 20 gm |
| Magnesium stearate | 2 gm |

The ibuprofen (finely divided by means of an air micronizer) is added to the other finely powdered ingredients, mixed thoroughly and then encapsulated in the usual manner.

The foregoing capsules are useful for remission induction of acute myelogenous leukemia in adults by the administration of 1 capsule 3 times a day for 2 days, followed by 6 hours of drug free interval, followed by 12 hours of administration of single cytotoxic agent and followed with 18 hours of drug free interval. The foregoing cycles are repeated until complete remission occurs.

Using the procedure above, capsules are similarly prepared containing ibuprofen in 100, 200, and 400 mg amounts by substituting 100, 200, and 400 gm of ibuprofen for the 600 gm used above.

Example 12 Soft Gelatin Capsules

One-piece soft gelatin capsules for oral use, each containing 600 mg of ibuprofen (finely divided by means of an air micronizer) are prepared by first making a paste of the compound in 1 ml of corn oil to render the material capsulatable and then capsulating in the above manner.

The foregoing capsules are used for treating acute myelogenous leukemia in adults by the administration regimen of Example 1.

Example 13  Tablets

One thousand tablets, each containing 400 mg of ibuprofen are prepared from the following types and amounts of ingredients:

| | |
|---|---|
| Ibuprofen | 400 gm |
| Lactose | 75 gm |
| Corn starch | 50 gm |
| Magnesium stearate | 4 gm |
| Light liquid petrolatum | 5 gm |

The ibuprofen (finely divided by means of an air micronizer) is added to the other ingredients and then thoroughly mixed and slugged. The slugs are broken down by forcing through a number sixteen screen. The resulting granules are then compressed into tablets, each tablet containing 400 mg of ibuprofen.

The foregoing tablets are used in remission induction of acute myelogenous leukemia by administering 2 tablets orally every 6 hours for 18 hours, followed by a drug free interval of 6 hours after which 5-azacytidine is administered parenterally for 12 hours. After this a drug free interval of 18 hours is followed by another cycle of ibuprofen and the 5-azacytidine. The number of cycles administered is determined by the status of bone marrow during the 18 hours drug free interval. When complete remission occurs ibuprofen is administered daily, at a dose of 2000 mg per day until 2 days prior to recommencement of the administration of cytotoxic agent used for maintenance chemotherapy.

Example 14  Oral Suspension

One thousand ml of an aqueous suspension for oral use, containing in each teaspoonful (5 ml) dose, 200 mg of ibuprofen, aluminum salt is prepared from the following types and amounts of ingredients:

| | |
|---|---|
| Ibuprofen, aluminum salt micronized | 40 gm |
| Citric acid | 2 gm |
| Benzoic acid | 1 gm |
| Sucrose | 700 gm |
| Tragacanth | 5 gm |

Lemon oil                                    2 gm

Deionized water, q.s.              1000 ml

The citric acid, benzoic acid, sucrose, tragacanth and lemon oil are dispersed in sufficient water to make 850 ml of suspension. The ibuprofen aluminum salt (finely divided by means of an air micronizer) is stirred into the syrup until uniformly distributed. Sufficient water is a added to make 1000 ml.

The oral composition so prepared is useful in remission maintenance by the administration of 4 teaspoonful twice a day.

Example 15

A sterile aqueous solution for parenteral (i.v.) injection, containing in 300 ml, 1 gram of ibuprofen, sodium salt is prepared from the following types and amounts of ingredients:

Ibuprofen, sodium salt              1 gm

Water for injection, q.s.        300 ml

To the sterile solution is added sterilized ibuprofen, sodium salt and filled into sterile containers sealed.

The composition so prepared is useful for treating acute myelogenous leukemia in children by administration of 300 ml every 12 hours for 2 days, followed by drug free interval of 6 hours, followed by an induction course of treatment with 5-azacytidine until remission is induced. When remission is induced ibuprofen is given orally on a daily basis during remission.

Example 16  Hard Gelatin Capsules

One thousand two-piece hard gelatin capsules for oral use, each capsule containing 400 mg of ibuprofen are prepared from the following types and amounts of ingredients:

Ibuprofen                           400 gm

Lactose                             100 gm

Corn starch                          20 gm

Talc                                 20 gm

Magnesium stearate                    2 gm

The ibuprofen (finely divided by means of an air micronizer) is added to the other finely powdered ingredients, mixed thoroughly and then encapsulated in the usual manner.

The foregoing tablets are used in remission maintenance of chronic myelogenous leukemia in adults by administering 1600 mg/day commencing upon termination of treatment with cytotoxic agents and recovery of platelet count to 100,000/cmm. and terminating 2 days prior to recommencing treatment with cytotoxic agents.

Using the procedure above, capsules are similarly prepared containing ibuprofen in 50, 100, and 200 mg amounts by substituting 50, 100, and 200 gm of ibuprofen for the 400 gm used above.

Example 17  Soft Gelatin Capsules

One-piece soft gelatin capsules for oral use, each containing 100 mg of ibuprofen (finely divided by means of an air micronizer) are prepared by first suspending the compound in 0.5 ml of corn oil to render the material capsulatable and then capsulating in the above manner.

The foregoing capsules are used in remission maintenance of chronic myelogenous leukemia by administering 1600 mg/day commencing upon termination of treatment with cytotoxic agents and recovery of platelet count to 100,000/cmm. and terminating 2 days prior to recommencing treatment with cytotoxic agents.

Example 18  Tablets

One thousand tablets, each containing 400 mg of ibuprofen are prepared from the following types and amounts of ingredients:

| Ibuprofen | 400 gm |
| Lactose | 75 gm |
| Corn starch | 50 gm |
| Magnesium stearate | 4 gm |
| Light liquid petrolatum | 5 gm |

The ibuprofen (finely divided by means of an air micronizer) is added to the other ingredients and then thoroughly mixed and slugged. The slugs are broken down by forcing through a number sixteen screen. The resulting granules are then compressed into tablets, each tablet containing 400 mg of ibuprofen.

The foregoing tablets are used in remission maintenance of chronic myelogenous leukemia by administering 1600 mg/day commencing upon termination of treatment with cytotoxic agents and recovery of platelet count to 100,000/cmm. and terminating 2 days prior to recommencing treatment with cytotoxic agents.

Example 19  Oral Suspension

One thousand ml of an aqueous suspension for oral use, containing in each teaspoonful (5 ml) dose, 200 mg of ibuprofen, aluminum salt is prepared from the following types and amounts of ingredients:

| Ibuprofen, aluminum | |
|---|---|
| salt micronized | 40 gm |
| Citric acid | 2 gm |
| Benzoic acid | 1 gm |
| Sucrose | 700 gm |
| Tragacanth | 5 gm |
| Lemon oil | 2 gm |
| Deionized water, q.s. | 1000 ml |

The citric acid, benzoic acid, sucrose, tragacanth and lemon oil are dispersed in sufficient water to make 850 ml of suspension. The ibuprofen aluminum salt (finely divided by means of an air micronizer) is stirred into the syrup until uniformly distributed. Sufficient water is added to make 1000 ml.

The oral composition so prepared is useful in remission maintenance by the administration of 4 teaspoonful twice a day.

Example 20

A sterile aqueous solution for parenteral (i.v.) injection, containing in 300 ml, 1 gram of ibuprofen, sodium salt is prepared from the following types and amounts of ingredients:

| Ibuprofen sodium salt | 1 gm |
|---|---|
| Water for injection, q.s. | 300 ml |

To the sterile solution is added sterilized ibuprofen, sodium salt and filled into sterile containers sealed.

The composition so prepared is useful for treating acute myelogenous leukemia in children by administration of 300 ml every 12 hours for 3 days, followed by 1 day of drug free interval, folowed by an induction course of treatment with cytotoxic agent until remission is induced. When remission is induced ibuprofen is given orally on a daily basis during remission of 600 mg/day until 2 days prior to commencement of administration of cytotoxic agents used for maintenance chemotherapy.

Example 21

Following the procedure of the proceeding Examples 1 through 20, inclusive, compositions are similarly prepared substituting equimolar amounts of the ester, e.g., methyl, ethyl, isopropyl, octyl, or salt, e.g., sodium, potassium ammonium, for the compound of the examples.

CLAIMS

1.    A compound which is flurbiprofen or ibuprofen, or a $C_{1-8}$ alkyl ester or a pharmacologically acceptable salt thereof, for use in the treatment of acute or chronic myelogenous leukemia.

2.    A compound as claimed in claim 1, for use in the case where the subject having leukemia is to be subsequently treated with a cytotoxic agent.

3.    A composition which comprises a compound as defined in claim 1 and a cytotoxic agent, for consecutive administration in the treatment of acute or chronic myelogenous leukemia.

4.    A package  which comprises a plurality of unit dosages of a compound as defined in claim 1 and a plurality of unit dosages of a cytotoxic agent.

5.    A compound or composition according to any preceding claim, in which the compound is flurbiprofen.

6.    A compound or composition according to any of claims 1 to 4, in which the compound is ibuprofen.

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | "DICTIONARY OF ORGANIC COMPOUNDS", 4th Edition, Tenth and Cumulative Supplement, 1973, Eyre & Spottiswoode Publishers Ltd., London (GB); *Page 553, entry "Ibuprofen"* | 1-5 | A 61 K 31/19 // C 07 C 57/30 C 07 C 57/58 |
| X | "DICTIONARY OF ORGANIC COMPOUNDS", 4th Edith, Fifteenth and Cumulative Supplement, 1978, Eyre & Spottiswoode Publishers Ltd., London (GB); *Page 402, entry "Flurbiprofen"* | 1-5 | |

TECHNICAL FIELDS
SEARCHED (Int. Cl. ³)

C 07 C 57/00

The present search report has been drawn up for all claims

| Place of search THE HAGUE | Date of completion of the search 28-04-1983 | Examiner KLAG M.J. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82